# EUROPÄISCHE PATENTSCHRIFT

(11) **EP 2 670 365 B1**
(45) Veröffentlichungstag und Bekanntmachung des Hinweises auf die Patenterteilung: **11.05.2016**
(21) Anmeldenummer: 12702754.8
(22) Anmeldetag: 25.01.2012
(51) Int. Cl.: A61F 5/30, A61F 13/06

(54) **VERBESSERTE PATELLAPELOTTE**
IMPROVED PATELLA PELOTTE
PELOTE ROTULIENNE AMÉLIORÉE

(30) Priorität: 31.01.2011 DE 102011010827
(43) Veröffentlichungstag der Anmeldung: 11.12.2013
(73) Patentinhaber: Bauerfeind AG, 07937 Zeulenroda-Triebes (DE)
(72) Erfinder: HESS, Heinrich, 66271 Kleinblittersdorf (DE); SCHEUERMANN, Rainer, 24223 Raisdorf (DE); BAUERFEIND, Hans, B., 07937 Zeulenroda-Triebes (DE); MARX, Oliver, 08056 Zwickau (DE)
(74) Vertreter: Wohlfahrt, Jan Günther
(86) Internationale Anmeldenummer: PCT/EP2012/000338
(87) Internationale Veröffentlichungsnummer: WO 2012/104037

(56) Entgegenhaltungen:
- DE-A1- 3 637 879
- DE-U1- 9 300 600
- DE-U1- 29 803 103
- US-A- 6 149 616

## Beschreibung

Die Erfindung betrifft medizinische Hilfsmittel zur Unterstützung der Funktion des Kniegelenks, besonders Kniegelenksorthesen mit die Patella des Kniegelenks umgreifender Pelotte. Die Erfindung stellt eine verbesserte Patellapelotte bereit, die zusätzliche bewegungsunterstützende und prophylaktische/therapeutische Funktionen besitzt.

Kniegelenksorthesen mit einem ringförmigen oder halbringförmigen Pelottenkissen, das die Patella des Kniegelenks im angelegten Zustand der Orthese umschließt, sind bekannt. Solche Orthesen können bekanntermaßen als strumpf- oder schlauchförmige elastische Gestricke ausgebildet sein. In dem Gestrick ist im Bereich der Patella eine Pelotte eingesetzt. Die Pelotte stützt die Patella und fixiert diese vor allem in Verbindung mit der durch das elastische Gestrick vermittelten Druckbeaufschlagung in physiologisch korrekter Position im Gelenk. Bekannte Pelotten sind aus einem elastischen Werkstoff wie Silikonkautschuk oder Polyurethan oder ähnlichen Werkstoffen gefertigt.

DE 29 803 103 U1 offenbart eine Kniebandage mit einer schlauchförmigen Hülle aus elastischem, dehnbarem Textilmaterial, die eine den Patellabereich bogenförmig umschließende und sich nach unten öffnende, C-förmige Pelotte aufweist.

US 6,149,616 A offenbart eine Bandage für das Kniegelenk aus einem elastischen Bandagenstoff in Schlauchform mit einem Einsatz aus Wellengestrick.

DE 9 300 600 U1 offenbart eine Kniepelotte aus einem flexiblen Kunststoffmaterial zum Einsatz in einer Knie-Orthese aus einem ringförmigen Körper.

Unterhalb des Kniegelenks, besonders im vorderen Kniekompartiment, liegt zu beiden Seiten der Kniescheibe ein infrapatellarer Fettkörper, der sogenannte "Hoffasche Fettkörper". Unter bestimmten physiologischen und hormonellen Voraussetzungen kommt es zu einer Hyperplasie des Fettkörpers und gegebenenfalls zu einer Hypotrophie mit dauerhaftem Ersatz der Fettzellen durch kollagenes Bindegewebe. In solchen Fällen kommt es bei Kniebeugung und Kniestreckung zu einem signifikanten Druckanstieg in dem Fettkörper, der mit einer schmerzhaften Bewegungsbehinderung einhergeht. Der infrapatellare Fettkörper dient im gesunden Organismus der Unterstützung der Gelenkfunktion, indem er primär Volumen in den Gelenkzwischenräumen füllt, um in jedem Bewegungszustand einen völlig kongruenten Schluss zwischen den Gelenkelementen von Tibia, Femur und Patella, besonders den Femurkondylen, den Meniskusvorderhörnern und der Tibiabasis, zu gewährleisten. Weiter wird dem infrapatellaren Fettkörper eine Weichteilbettung von Sehnen, besonders der Patellasehne, zur Stabilisierung der Patella in Streckstellung, sowie die mechanische Dämpfung der Lastübertragung und die Positionierung der Patella zugeschrieben. In bestimmten Zuständen kann es zu einer Volumenreduzierung und Atrophie der Fettkörper kommen. Die unterstützende Funktion ist dann nicht mehr gewährleistet. Daneben sind die infrapatellaren Fettkörper auch unmittelbar an der Schmerzentstehung im Kniegelenk beteiligt. Untersuchungen zeigen, dass die Fettkörper selbst mit Drucksensoren und Schmerzleitungsfasern innerviert sind.

Die Erfindung hat sich zur Aufgabe gestellt, das Anwendungsgebiet gattungsgemäßer Kniegelenksbandagen beziehungsweise Orthesen mit eingesetzter Patellapelotte zu erweitern, sodass im Zusammenhang mit dem infrapatellaren Fettkörper des Kniegelenks stehende pathologische Zustände, insbesondere Schmerzzustände und Bewegungseinschränkungen, prophylaktisch und therapeutisch behandelt werden können.

Das der Erfindung zugrunde liegende technische Problem wird gelöst durch die Bereitstellung einer neuartig gestalteten Pelotte für eine Kniegelenksorthese. Diese weist eine an sich bekannte ringförmige oder halbringförmige Basis auf, welche im angelegten Zustand der Orthese oder Bandage die Patella umgreifen oder umschließen kann. Erfindungsgemäß sind an dieser Basis distal zum Gelenk weisende, insbesondere paarige, Vorsprünge ausgebildet. Diese sind derart ausgestaltet, dass sie im angelegten Zustand der Orthese oder Bandage jeweils im Bereich der infrapatellaren Fettkörper des Kniegelenks anordenbar sind und so auf diese jeweils Druck ausüben können. Die Vorsprünge sind besonders als Noppen oder Finger ausgebildet, die aus dem Pelottenring oder -bogen herausragen oder ansetzen. Die Pelotte weist an ihrer Basis distal zusätzlich seitliche, insbesondere flügelförmige, Vorsprünge auf. Diese sind derart ausgestaltet, dass sie im angelegten Zustand der Orthese oder Bandage, insbesondere distal der Patella, mit den infrapatellaren Gelenkspalten eingreifen können.

Die distalen Vorsprünge verdrängen in bevorzugter Ausführung das distale Fettgewebe vor allem nach oben, so dass sich die Vorsprünge erfindungsgemäß bevorzugt zumindest teilweise unter den unteren Patellapol schieben und diesen stabilisieren können. Dadurch kann sich die Patella von den Gelenkknochen des Kniegelenks abheben, und eine reibende Verbindung und damit weiterer Verschleiß und Schmerzen vermindert oder verhindert werden.

Die Pelotte ist in ihrer Grundform (Pelottenbasis) besonders ringförmig ausgebildet und umschließt die Patella. In einer anderen besonderen Ausgestaltung ist die Pelotte hufeisenförmig, das heißt halbringförmig, ausgebildet und umgreift die Patella zumindest distal.

Die neuartige Pelotte wird in an sich bekannter Weise bevorzugt in einer elastischen Gestrickorthese eingesetzt. Über die Gestrickorthese wird die Pelotte an das Kniegelenk im Bereich der Patella angepresst. Die distalen Vorsprünge entfalten ihre Wirkung vor allem in Verbindung mit dem elastischen Grundgestrick der Bandage und treten durch die Bewegung mit diesem in Wechselwirkung.

Erfindungsgemäß weist die Pelotte an ihrer Basis distal zusätzlich seitliche Vorsprünge auf. Diese sind derart ausgestaltet, dass sie im angelegten Zustand der Orthese oder Bandage, insbesondere distal der Patella, in seitliche Gelenkspalte eingreifen können.

Besonders die Kombination der im Bereich des infrapatellaren Fettkörpers lokalisierbaren Vorsprünge mit den distalen seitlichen Vorsprüngen ergibt sich überraschend eine adäquate physiomechanische Beeinflussung des infrapatellaren Gelenkapparats, besonders der infrapatellaren Fettkörper durch die neuartige Pelotte. Die Erfinder fanden überraschend, dass sich durch eine durch diese erfindungsgemäßen Vorsprünge der Pelotte vermittelte Druckbeaufschlagung des infrapatellaren Gelenkapparats, insbesondere des infrapatellaren Fettkörpers, das an sich bekannte vordere Knieschmerzsyndrom spezifisch behandeln lässt, dass sich die Gelenkfunktion unterstützende Funktion des Fettkörpers verbessern lässt und sich Ödeme oder eine Hyperplasie oder Hypertrophie der Fettkörper prophylaktisch und therapeutisch behandeln lassen.

Daneben ist bevorzugt zusätzlich vorgesehen, dass die erfindungsgemäßen distalen zum Gelenk gewandten Vorsprünge so mit dessen anatomischen Strukturen in Eingriff kommen und so die exakte anatomische Positionierung der Bandage unterstützen.

Die erfindungsgemäßen distalen Vorsprünge der Pelotte erlauben außerdem eine bessere Positionierung der Pelotte über der Patella. Die Vorsprünge "rasten" gleichsam in die distalen Vertiefungen des Kniegelenkspalts ein und stabilisieren die Position der Pelotte an der Patella und sichern so die Wirkung der Gelenksbandage.

Die Pelotte ist bevorzugt aus einem dauerelastischen Werkstoff, insbesondere Silikonkautschuk oder Polyurethan, gebildet. Die Erfindung ist jedoch nicht auf diese Werkstoffe beschränkt. Der Fachmann kennt gleichermaßen geeignete Werkstoffe. Dabei ist insbesondere das physikalisch-mechanische Verhalten des Werkstoffs, vor allem der Elastizitätsmodul, an das Weichteilgewebe des Kniegelenks angepasst.

Die Erfindung sieht in besonderer Ausgestaltung vor, dass die Höhe und/oder die Nachgiebigkeit der zu dem Fettkörper hin gerichteten distalen Vorsprünge der Pelotte individuell gestaltbar ist, um die Druckbeaufschlagung auf die Fettkörper individuell zu steuern. Dies kann im Rahmen eines Therapieplans oder zur Verwirklichung verschiedener prophylaktischer oder therapeutischer Ziele wünschenswert sein. Die Erfindung sieht dazu beispielsweise vor, dass die erfindungsgemäße Pelotte lösbar mit der Kniegelenksorthese oder Bandage verbunden ist, und aus dieser zum Zwecke des Einstellens oder Anpassens der Höhe oder der Materialeigenschaften, besonders der Nachgiebigkeit, das heißt Shore-Härte, der Vorsprünge herausnehmbar und anschließend wieder einsetzbar ist. Dabei ist besondern vorgesehen, dass die distalen Vorsprünge an der Pelottenbasis zur Einstellung der Wirkung der Pelotte auf den Fettkörper auswechselbar sind, vor allem dass Vorsprünge mit anderen Höhen oder anderen mechanischen Eigenschaften einsetzbar sind. Alternativ kann vorgesehen sein, dass die gesamte Pelotte, Basis und Vorsprünge, als integrales einstückiges Element ausgebildet ist und zur Einstellung der Wirkung auf den Fettkörper als Ganzes gegen eine Pelotte mit anderen mechanischen Eigenschaften, besonders Materialeigenschaften, auswechselbar ist.

Gegenstand der Erfindung ist auch eine Kniegelenksorthese oder -bandage, welche die erfindungsgemäße Pelotte enthält. Diese ist besonders als Gestrickorthese mit eingelegter erfindungsgemäßen Pelotte ausgestaltet.

Gegenstand der Erfindung ist auch die Verwendung der erfindungsgemäßen Pelotte mit den daran ausgebildeten Vorsprüngen zur Verbesserung und/oder zur Sicherung der Positionierung der Gelenksorthese am Körpergelenk.

Die Erfindung wird anhand der nachfolgenden Figuren näher beschrieben, ohne dass die dort dargestellten Ausführungsformen der Erfindung beschränkend zu verstehen sind.

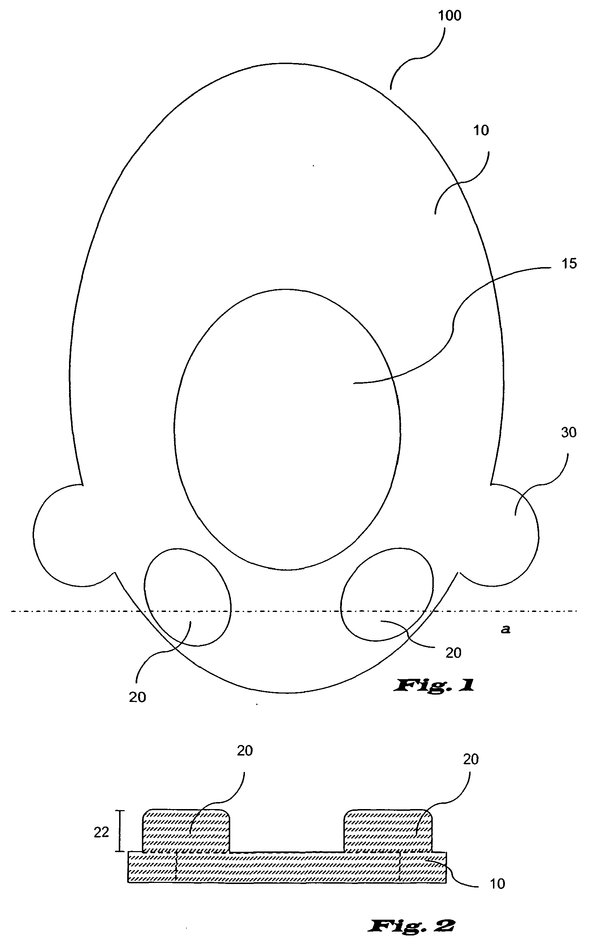

Figur 1 zeigt eine Ausgestaltung der erfindungsgemäßen Pelotte (100) mit einer ringförmigen Basis (10), welche die in der Ausnehmung (15) lokalisierte Patella umschließt. Die Basis (10) weist erfindungsgemäß paarige zum Gelenk weisende noppenförmige Vorsprünge (20) auf. Zusätzlich weist die Pelotte distale seitliche flügelförmige Vorsprünge (30) auf.
In Figur 2 ist ein Querschnitt an der Schnittlinie a (Figur 1) dargestellt. Die noppenförmigen Vorsprünge (20) ragen in einer vorbestimmbaren Höhe (22) aus der Basis (10) heraus in Richtung des Gelenks.

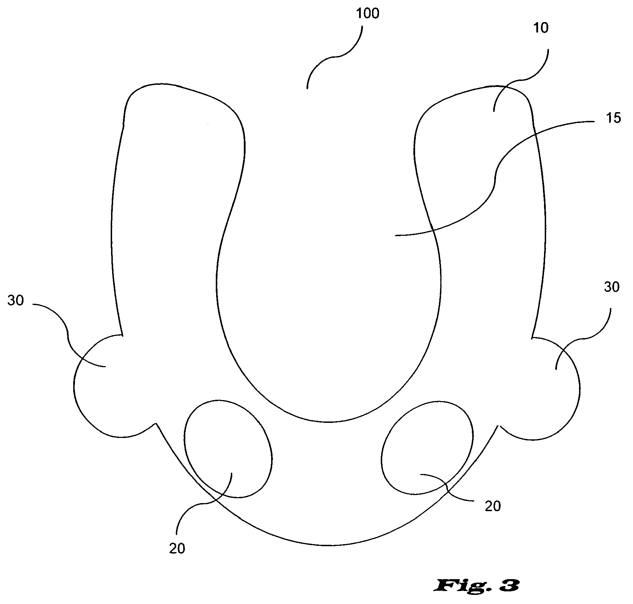

Figur 3 zeigt eine alternative Ausgestaltung der Pelotte (100), welche die in der Ausnehmung (15) lokalisierte Patella halbkreis- oder hufeisenförmig distal umgreift.

## Patentansprüche

1. Pelotte (100) für eine Kniegelenksorthese mit einer ringförmigen oder halbringförmigen Basis (10) zum Umgreifen der Patella, wobei an der Basis distal zum Gelenk weisende Vorsprünge (20) ausgebildet sind, wobei die Vorsprünge (20) im angelegten Zustand der Pelotte im Bereich der infrapatellaren Fettkörper anordenbar sind und auf diese Druck ausüben können, und wobei die Basis (10) zusätzlich seitliche flügelförmige Vorsprünge (30) aufweist, die im angelegten Zustand der Pelotte mit den infrapatellaren Gelenkspalten in Eingriff kommen können.

2. Pelotte nach Anspruch 1, wonach die Vorsprünge (20) für die Wirkung auf den Fettkörper in ihren mechanischen Eigenschaften einstellbar oder auswechselbar sind.

3. Verwendung der in einem der vorstehenden Ansprüche charakterisierten Pelotte in eine Kniegelenksorthese zur Verbesserung und/oder Sicherung der Positionierung der Kniegelenksorthese an dem Kniegelenk.

4. Kniegelenksorthese, enthaltend die Pelotte (100) nach einem der Ansprüche 1 oder 2.

5. Kniegelenksorthese nach Anspruch 4, die eine elastische Gestrickorthese ist.

## Claims

1. Pelotte (100) for a knee-joint orthosis, said pelotte having an annular or semi-annular base (10) for engaging around the patella, wherein projections (20) configured to face the joint are formed distally on the base, wherein the projections (20), in a fitted state of the pelotte, are configured to be located in the area of infrapatellar fat bodies and are adapted to exert pressure on said fat bodies, and wherein the base (10) additionally has lateral wing-shaped projections (30), which, in the fitted state of the pelotte, are configured to come into engagement with the infrapatellar joint spaces.

2. Pelotte according to claim 1, wherein the projections (20) are adjustable in terms of their mechanical properties or are exchangeable in order to act on the fat bodies.

3. Use of the pelotte **characterized in** any of the preceding claims in a knee-joint orthosis for improving and/or securing positioning of the knee-joint orthosis on the knee joint.

4. Knee-joint orthosis, comprising the pelotte (100) according to any one of claims 1 or 2.

5. Knee-joint orthosis according to claim 4, which is an elastic knitted orthosis.

## Revendications

1. Pelote (100) pour une genouillère avec une base circulaire ou semi-circulaire (10) pour envelopper la rotule, dans laquelle des saillies (20) tournées de manière distale vers l'articulation sont réalisées sur la base, dans laquelle les saillies (20) peuvent être disposées à l'état appliqué de la pelote dans la région des corps adipeux infra-patellaires et peuvent exercer de la pression sur ceux-ci, et dans laquelle la base (10) présente en outre des saillies latérales en forme d'aile (30) qui peuvent s'engager à l'état appliqué de la pelote avec les espaces articulaires infra-patellaires.

2. Pelote selon la revendication 1, suivant laquelle les saillies (20) peuvent être réglées dans leurs propriétés mécaniques ou échangées pour l'action sur le corps adipeux.

3. Utilisation de la pelote caractérisée dans une des revendications précédentes dans une genouillère pour l'amélioration et/ou la sécurisation du positionnement de la genouillère sur l'articulation du genou.

4. Genouillère contenant la pelote (100) selon une des revendications 1 ou 2.

5. Genouillère selon la revendication 4, qui est une orthèse tricotée élastique.
